(19) Europäisches Patentamt

European Patent Office

Office européen des brevets

(11) **EP 1 152 349 A1**

(12) **EUROPEAN PATENT APPLICATION**

(43) Date of publication:
07.11.2001 Bulletin 2001/45

(51) Int Cl.⁷: **G06F 17/30**

(21) Application number: **00109664.3**

(22) Date of filing: **06.05.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Deutsches Krebsforschungszentrum Stiftung des öffentlichen Rechts**
**69120 Heidelberg (DE)**

(72) Inventors:
• **Spang, Rainer**
**69120 Heidelberg (DE)**
• **Stoye, Jens**
**69124 Heidelberg (DE)**

(74) Representative: **Castell, Klaus, Dr.**
**Gutenbergstrasse 12**
**52349 Düren (DE)**

(54) **Method for aligning sequences**

(57) The invention is directed to a method for finding a relation between at least one candidate sequence and a number of prealigned seed sequences; wherein the sequences are defined by a consecutive string of characters, each of which belongs to a group of symbols including a gap symbol; with the following step: -Finding an optimal alignment between the candidate sequence and the prealigned seed sequences by determining for every position of the candidate sequence the best fit with a corresponding position of one of the seed sequences; characterised in that for determining the optimal individual alignment at each position under evaluation, the following parameters are used: -the match between the symbol of the candidate sequence and the symbol of the seed sequence at the positions under evaluation; -the benefit of introducing a gap as a new position into one of the two sequences under evaluation at a position under evaluation; and -the benefit of jumping from one seed sequence under evaluation to another seed sequence.

Fig. 1

EP 1 152 349 A1

**Description**

This invention is directed to a method for aligning sequences.

Whenever the question arises if two or more sequences of characters which are selected from a group of symbols show similarities throughout them, a method must be chosen to compare the two or more sequences, and a score must be determined in order to quantify such similarities. Examples for such sequences might be textual strings and numerical patterns. A field of particular importance for finding such similarities is molecular biology, where nucleotide and peptide sequences must be compared.

E.g., the determination of the function of proteins is among the most fundamental problems in molecular biology. In principle, this task requires elaborate experimental studies for each protein in question. The genome-era confronts the researchers with a large number of protein and nucleotide sequences of unknown function. Scientists now strive for a large scale understanding of protein function. In view of the new sequence data produced by the genome projects, it is crucial to ask for fast and easy methods of functional analysis.

Roughly speaking, proteins with similar function descend from common ancestors. They have been randomly altered by mutation events; however, natural selection has extinguished those mutant molecules that do not preserve the protein's function. At the level of sequences, common function is often reflected in terms of conserved regions of the sequences. The standard approach is to compare unknown sequences to proteins with known function. Bioinformatic scientists have contributed to this project in that they have developed large assortments of computer based methods for extracting information from molecular sequences via sequence comparison, characterisation, and classification.

First, a short introduction into the concepts of aligning two sequences, e.g. protein sequences, will be given. It is well known that the concept of dynamic programming is well suited for many sequence alignment problems. For example, the Smith-Waterman algorithm (Smith and Waterman, J. Mol. Biol., Vol. 147, pgs. 195-197, 1981) is one of the most widely used procedures for aligning pairs of molecular sequences. In the following, a brief review of the concept of dynamic programming is given in the simpler case of local pair wise alignments.

The present example deals with finding and aligning similar segments in a pair of sequences. This requires a formal definition of sequence similarity, which is usually done by introducing a score matrix w which provides a score for every pair of amino acids or nucleotides. In general, similar or identical amino acids are assigned positive scores, whereas dissimilar amino acids obtain negative scores. The expectation value of the score must be negative in order to ensure that the alignments are local. In addition, the gap positions in an alignment must be scored. Presently, this is done by penalising each position in a gap by the same constant value, called gapcost. Given a local alignment, one obtains an alignment score by summing up all similarity scores of positions where two amino acids are aligned to each other, and then subtracting all gap costs. The problem resides in constructing a local alignment with an optimal similarity score among all possible local alignments of the two sequences.

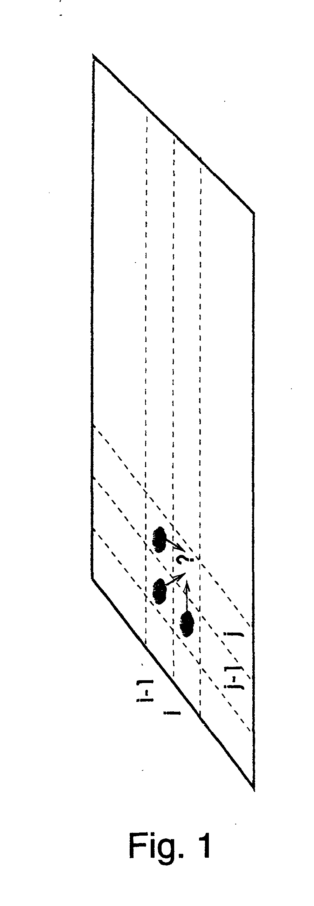

There is a simple dynamic programming algorithm for solving this optimisation problem rigorously. Assume there are given two sequences $S = s_1,...,s_n$ and $T = t_1,...,t_m$ of length n and m, respectively. The algorithm runs on an (n+1) x (m+1) matrix D, called the edit matrix. For 1 i n, 1 j m, cell D(i,j) holds the score of the best local alignment of the sequences that ends with the positions $s_i$ and $t_j$. The leftmost column and the top row of D are initialised by D(i, O) = D(O, j) = O. Figure 1 illustrates how the remainder of D is computed recursively by determining the maximum for D(i,j) based on the following score values:

0,

$$D(i-1,j-1) + w(s_i, t_j)$$

$$D(i-1,j) - gapcost$$

$$D(i,j-1) - gapcost$$

The optimal local alignment score is the largest entry in the edit matrix, $D(i_{max},j_{max})$.

This algorithm runs in time O(nm) and uses O(nm) space to store the matrix D. The alignment itself is obtained by tracing back through the matrix from $D(i_{max}, j_{max})$ following the choices that were made in the maximisation, until a cell with entry 0 is reached. It is a standard technique, however, to reduce the memory usage to O(n + m) by a method introduced by Hirschberg (Comun. ACM, Vol. 18, pgs. 341-343, 1975), and extended to the con-text of local alignment by Huang et al. (CABIOS, Vol. 6, pgs. 373-381, 1990), at the expense of essentially only doubling the computation time. If being only interested in the score of the best local alignment, however, it is much easier to reduce space.

Assuming a row-wise computation order, to compute entries in e.g. row i, only values from rows i and i - 1 are needed. Hence, it suffices to store only two neighbouring rows at any time of the algorithm which reduces space requirements to O(m).

Hitherto, the algorithm in the case of linear gap costs was described where the penalty for a gap is proportional to the length of the gap. It is widely acknowledged that a long gap should be penalised less than two shorter gaps of the same length in total. Since computationally cheap, so-called affine gap costs are in common use. Here, the initiation of a gap is penalised by a high value "gapinit", and each additional character of the gap is penalised by another, usually smaller value "gapext". Based on ideas first published by Gotoh (Journal Mol. Biol., 162, pgs. 705-708; 1982), Huang et al. show how to incorporate affine gap costs in local alignments without an overhead in computational complexity.

In the following, the concept of comparing multiple sequences will be introduced, with an example selected from molecular biology.

Classification of proteins on the basis of common conservation patterns is a standard approach in computational biology. Families of proteins with known functions are grouped together and then, given an uncharacterised protein or nucleotide sequence, the question arises if this sequence fits into one of the known families. In this setting, several methods have been developed, like templates (Taylor, Journal of Molecular Biology, Vol. 188, pgs 233-258, 1986), profiles (Gribskov et al., Proc. Nat. Acad. Sci. US, Vol. 84, pgs. 4355-4358, 1987; Luthy et al., Protein Sci., Vol. 3, pgs. 130-146, 1994) hidden Markov models (Krogh et al., Journal of Molecular Biology, Vol. 235, pgs. 1501-1531, 1994; Baldi et al., Proc. Nat. Acad. Sci. US, Vol. 91, pgs. 1509-1063, 1994; Eddy et al., Journal Comp. Biol., Vol. 2, pgs. 9-23, 1995), Bayesian models (Liu et al., J. Am. Stat. Assoc., Vol. 90, pgs. 1156-1170, 1995) and discriminative approaches like the Fisher kernel method (Jaakkola et al., in Proceedings of the Seventh International Conference on Intelligent Systems, ISMB 99, pgs. 149-158, 1999). The first four methods have a common rationale: First the information common to all sequences of a family is extracted, and then the unknown sequence is analysed for existence of these family-specific features. This approach generally requires a "summary" of the protein family which can be derived from a multiple alignment.

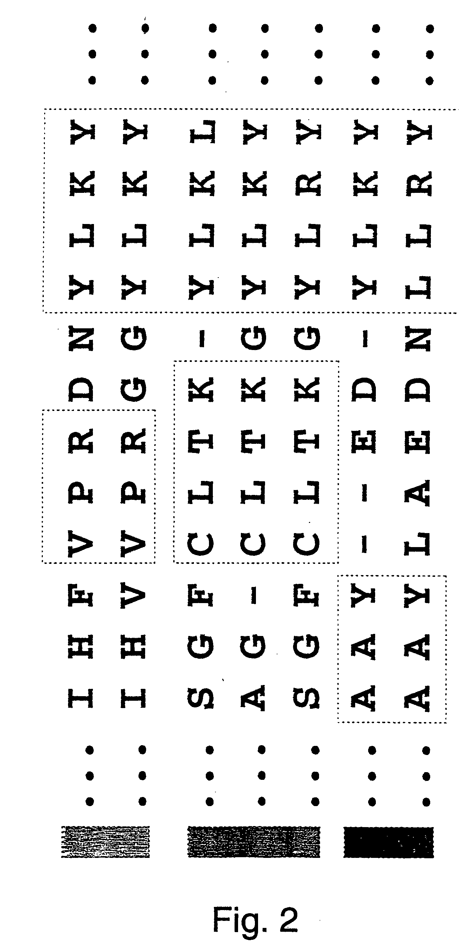

Figure 2 shows some columns of a multiple alignment that exhibits a typical problem with the approach detailed above.

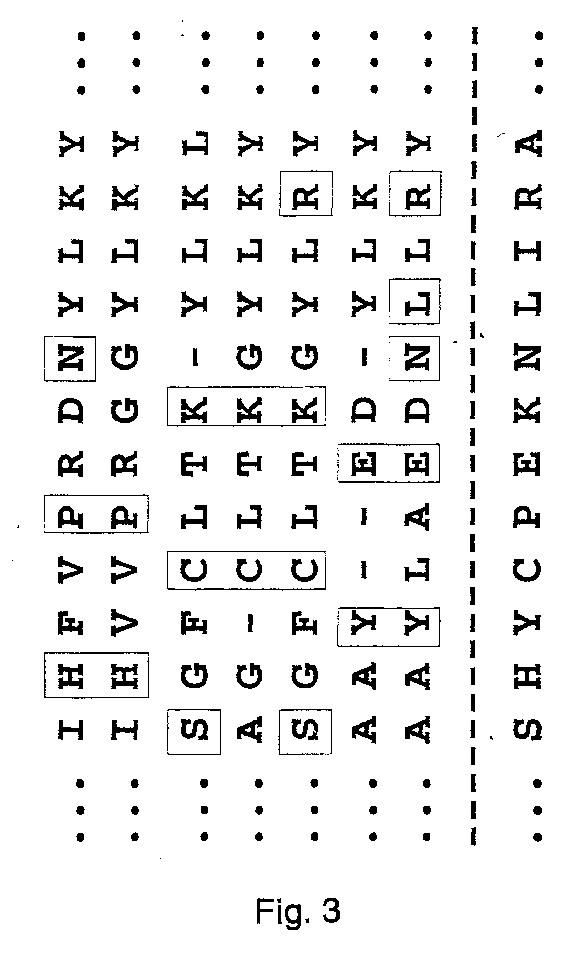

As can be seen from Fig. 2, the 7 sequences can be subdivided into 3 subfamilies, as indicated by the shaded bars on the left of Fig. 2. A first approach for summarising the information in the alignment is to proceed column by column: In the first column. either an 'I', an 'S' or an 'A', can be observed, in the second column 'H'.'G' or 'A ' etc. When deciding whether a candidate sequence fits into this family, it can be aligned to the family and then position after position can be checked in order to find out whether the residue of the candidate sequence is identical or similar to one of the residues in the family alignment at this position. This "vertical view", i.e. comparing identical positions among the sequences of a multiple alignment, forms the basis for most sequence classification methods. So called profiles consist of column specific scores, representing the residual distribution in these columns. Essentially the same is true for the emission distributions of hidden Markov models and the product multinomial distributions of block motifs in the Bayesian alignment approach. However, there is more information contained in the alignment: In column 4 there is frequently a 'C', but if there is a C' at this position, it is part of the conserved pattern 'C L T K'. This information is obscured in a column based summary of the alignment. A horizontal view on the alignment, i.e. looking at several consecutive positions of a sequence and comparing those to other sequences, reveals this. The importance of this horizontal information becomes evident from Figure 3.

The alignment in Figure 3 is the same as in Figure 2. In addition, a candidate sequences is shown below the dashed line. Taking the "vertical" point of view, one would clearly deduce that the candidate sequence fits very well into the family, since for almost all residues of the candidate sequence, the same residue can be found several times among the family sequences at the same position. However, none of the individual sequences in the family are very similar to the candidate sequence, thus speaking against a membership in the family. The vertical view indicates a good fit of the candidate sequence to the family; however, this indication is based on a : "wild hopping" through the alignment, as is shown by the highlighted residues in Figure 3. This problem is aggravated when considering an alignment consisting of a large number of sequences from a divergent family. In such a case, there are many alternative residues for most sites and hence there is a very high probability of chance hits, due to the "hopping phenomenon". Hopping causes noise in sequence classification.

As can be recognised from the foregoing, there is still a need for improving the methods used for aligning multiple sequences. It is therefore the object of the present invention to overcome the "vertical" bias of hitherto known multiple sequence alignments.

This object is solved by the provision of a method for finding a relation between at least one candidate sequence and a number of prealigned seed sequences according to independent claims 1 and 2. Further advantageous features, aspects, and details of the invention will become evident from studying the dependent claims, the description, and the drawings.

The invention is accordingly directed to a method for finding a relation between at least one candidate sequence and a number of prealigned seed sequences; wherein the sequences are defined by a consecutive string of characters, each of which belongs to a group of symbols including a gap symbol; with the following step:

- Finding an optimal alignment between the candidate sequence and the prealigned seed sequences by determining for every position of the candidate sequence the best fit with a corresponding position of one of the seed sequences; characterised in that for determining the optimal individual alignment at each position under evaluation, the following parameters are used:

- the match between the symbol of the candidate sequence and the symbol of the seed sequence at the positions under evaluation;

- the benefit of introducing a gap as a new position into one of the two sequences under evaluation at a position under evaluation; and

- the benefit of jumping from one seed sequence under evaluation to another seed sequence.

Hence, in this aspect of the invention, the candidate sequence is compared to a whole set of seed sequences which had already been aligned, e.g. by a computerised method for aligning. For every position of the candidate sequence (i.e. a particular symbol occurring as the i-th character of the candidate sequence), the best fit with all seed sequences is found. Consideration is given to the mere match of symbols (are they similar or dissimilar?), the benefit of a so called gap, i.e. the introduction of a gap symbol at either the candidate sequence or one of the seed sequences, and, as particular feature of the invention, the switch from one seed sequence to another one, here called jumping. Based on all these factors, a decision is made which direction to take for the alignment of that particular position.

In a more mathematical aspect of the invention, it is directed to a method for finding a relation between at least one candidate sequence and prealigned seed sequences; wherein the sequences are defined by a consecutive string of characters, each of which belongs to a group of symbols including a gap symbol. This inventive method comprises the following steps:

- setting up a matrix containing in a first dimension the n positions for the characters of the candidate sequence, in a second dimension m positions for the characters of the seed sequences, and in a third dimension K positions for the prealigned seed sequences, wherein K is at least 2, and having $(n +1) \times (m +1) \times K$ cells $D(i,j,k)$ with $0 <=i <=n$, $0 <=j <=m$, and $1 <=k <=K$ for holding the score of the best sequence alignment that ends with position i of the candidate sequence and position j of seed sequence k;

- determining a total maximum score $D(i_{max}, j_{max}, k_{max})$ at every cell of the matrix by recursively computing each $D(i, j, k)$ by setting it as the maximum of the group of possible values given by:

- 0 (zero);

- $D(i-1,j-1,k) +w(s_i, t_{jk})$;

- $D(i-1,j,k)$ - gapcost;

- $D(i,j-1,k)$ - gapcost

- $D(i-1,j-1,k) +w(s_i, t_{jk})$ - jumpcost

- $D(i-1,j,k)$ - gapcost - jumpcost

- $D(i,j-1,k)$ - gapcost - jumpcost

wherein

$s_i$ designates the symbol at position i of the candidate sequence,

$t_{jk}$ designates the symbol at position j of seed sequence k;

w is a preset score for every pair wise combination of symbols s and t;

k'is any of K- 1 seed sequences except seed sequence k;

a gapcost is a cost for skipping to the next position of one of the two sequences without also stepping to the next position of the other sequence; and

a jumpcost is a cost for jumping to another seed sequence k.

Preferably, the contents for cells D(i, 0, k) and D(0, j, k) are initialised by setting the D terms as defined above, used for computing them, to zero. Hence, for the "starting" cells of a recursive alignment, the above expressions of the form D(i(-1),j(-1),k(')) are simply set to zero.

When the maximum scores have been determined, it will be necessary to find the optimal alignment which is comprised of the so-called reference sequences. This can preferably be done with the following additional step of the inventive process:

- obtaining the optimal alignment by tracking back through the matrix from the maximum score $D(i_{max},j_{max},k_{max})$ along the choices made in the step of maximising the score, until a cell with a value of 0 is reached.

By this approach, it can be guaranteed that all positions which individually have the best local alignment, will be elected for the overall alignment.

Instead of using a three dimensional monolithic matrix, the matrix may be comprised of K two dimensional edit matrices with cells $D_k(i, j)$, wherein $1 <=k <=K$. In the following, the terminology will adhere to a single, three dimensional matrix. It is however to be understood that also two dimensional matrices may be used instead without giving up on the gist of the invention. Then, the term "k" used throughout the formulas given, is not indicative of the third dimension, but merely gives a designator of one of the two dimensional matrices.

As has been explained above, the gapcost associated with introducing a gap into one of the sequences, should depend on whether the gap will be the first gap introduced at that position, or will be a follow up gap position already introduced at a previous position. Accordingly, the gapcost may have different values, one of which is used when a gap is first introduced into the alignment, and at least another one which is used when a gap is extended in the alignment.

Consideration should also be given to whether a gap should be introduced in the candidate sequence or one of the seed sequences, or whether a gap might be introduced in the candidate sequence reflecting a like gap in one of the seed sequences. Accordingly, for the total gapcost of a gap, one of the following values may be used:

- a first gapcost if a gap is to be introduced in either the candidate sequence or the seed sequence;

- a second gapcost if no gap is introduced in the candidate sequence, even though a gap already exists in the pre aligned sequence; and

- a gapcost of zero if a gap is introduced in the candidate sequence, provided that a corresponding gap already exists in the pre aligned sequence

The first gapcost may be comprised of a gapinit cost, if the gap position is the first one introduced at a particular position i or j of the sequence, and gapext costs, for every consecutive gap position introduced at the following positions of the sequence.

The second gapcost may be comprised of gapext costs for every gap position in a consecutive succession of gap positions in the pre aligned sequence.

There are several approaches for reducing the necessary computational effort and hence, the time scale required to calculate the alignment according to the invention. In general, an intermediate result is calculated which focuses the group of possible seed sequences to be chosen from to the best one at a specific position of the candidate sequence and the seed sequences. One such preferred approach is to find the best sequence at cell co-ordinates (i, j-1) (horizontal), (i-1,j) (Vertical), and (i-1,j-1) (diagonal), and thereby avoid the process of selecting from all possible sequences. This preferred method is therefore characterised in that the maximum values of

$$D(i-1,j-1,k')$$

$$D(i,j-1,k');$$

and

$$D(i-1,j,k')$$

are pre selected by determining the sequence $k_d$ with the maximum values of the cells $D(i-1,j-1)$, the sequence $k_v$ with the maximum value of the cells $D(i-1,j)$ and the sequence $k_h$ with the maximum value of the cells $D(i,j-1)$ for all sequences $k'$ by the formulas:

$$k_d = \arg \max_{k' \in \{1,\ldots,K'\}} \{D(i-1, j-1, k')\}$$

$$k_v = \arg \max_{k' \in \{1,\ldots,K\}} \{D(i-1, j, k')\}$$

$$k_h = \arg \max_{k' \in \{1,\ldots,K'\}} \{D(i, j-1, k')\}$$

A further simplification can arise when precalculating the affine gap costs and finding the sequence in vertical and horizontal direction which has the best score. The optimal affine gap costs may be calculated by the following additional steps:

- setting up two auxiliary matrices V and H containing in a first dimension n positions for the characters of the candidate sequence, in a second dimension m positions for the characters of the seed sequences, and in a third dimension K positions for the K pre aligned seed sequences, for holding the score of the best sequence alignment that ends with position i of the candidate sequence and position j of the seed sequence k;

- determining the sequence $k_V$ with the maximum values of the cells $V(i-1, j)$, and the sequence $k_H$ with the maximum value of the cells $H(i,j-1)$ for all sequences $k'$ by the formulas:

$$k_V = \arg \max_{k' \in \{1,\ldots,K\}} \{V(i-1, j, k')\}$$

$$k_H = \arg \max_{k' \in \{1,\ldots,K\}} \{H(i, j-1, k')\}$$

While the storage needs of this preferred embodiment increase due to the additional matrices required, the time for computing the scores will be reduced. The results may then be used for determining the score themselves by the additional step:

- determining a total maximum score $D(i_{max}, j_{max}, k_{max})$ at every cell of the matrix

by recursively computing each $V(i,j,k)$ by setting it as the maximum of the group of possible values given by:

$$D(i-1,j,k) - gapinit$$

$$V(i-1,j,k) - gapext$$

$$D(i-1,j,k_v) - gapinit - jumpcost$$

$$V(i-1,j,k_v) - gapext - jumpcost;$$

by recursively computing each H(i,j,k) by setting it as the maximum of the group of possible values given by:

$$D(i,j-1,k) - gapinit$$

$$H(i,j-1,k) - G(j,k)$$

$$D(i,j-1,k_h) - gapinit - jumpcost$$

$$H(i,j-1,k_H) - G(j, k) - jumpcost;$$

and
by recursively computing each D(i,j,k) by setting it as the maximum of the group of possible values given by:

- 0 (zero);

- $D(i-1,j-1,k) + w(s_i, A_{k,j})$;

- $D(i-1,j-1,k_d) + w(s_i, A_{k,j}) - jumpcost$;

- $V(i,j,k)$;

- $H(i,j,k)$;

- $D(i,j-i,k)$ if $A_{k,j} = gapchar$;

- $D(i,j-1,k_h) - jumpcost$ if $A_{k,j} = gapchar$

wherein $A_{k,j}$ designates the symbol at the j-th position of the seed sequence k under evaluation;
gapchar designates the symbol used for indicating a gap;
G(j, k) is 0 if $A_{k,j} = gapchar$ or gapext otherwise; and
gapext is the value used when a gap is extended.
In the following, the invention will be further detailed and explained. Reference will be taken to the drawings in which

Fig. 1, as described, shows a graphical representation of a known edit matrix;

Fig. 2, as described, shows an alignment performed by prior art methods;

Fig. 3 , as described, shows the alignment of Fig. 2, including a candidate sequence;

Fig. 4 is an example of a score matrix w;

Fig. 5 shows a graphical representation of the inventive, three dimensional edit matrix;

Fig. 6 depicts an example of introducing gaps in the candidate sequence when gaps are present in seed sequences.;

Fig. 7 is a graph illustrating the correlation between preset jumpcost values and the false positive counts.;

Fig. 8 is a graph illustrating the correlation between preset cutoff c and the number of successful searches in a total of 40 data sets;

Fig. 9 shows a list of correlated sequences when a hidden Markov model is used; and

Fig. 10 shows a list of correlated sequences when the inventive method is applied to a database.

The invention is essentially an extension of the dynamic programming approach outlined above to jumping alignments. Given a multiple alignment consisting of a few sequences which represent a protein family, and a database of other sequences, the question arises which of the database entries fit into this family. The inventive idea is to evaluate this fit by means of optimal jumping alignment scores. For the purposes of this invention, the multiple alignment that describes a protein family is referred to as "seed alignment" and each sequence that is compared to it is referred to as a "candidate sequence".

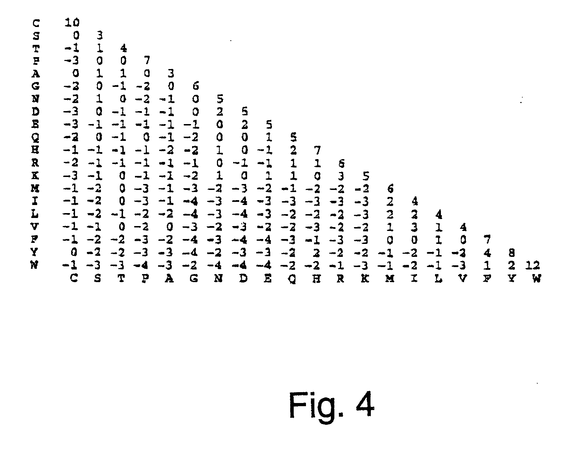

In order to avoid confusion, the inventive methods will be described stepwise, starting with a simple variant, and then adding more advanced features which improve its performance. First, linear gap costs will be discussed. The scoring scheme thus consists of the score matrix (w), the costs for a gap position (gapcost), and the penalty for jumping from one reference sequence to another (jumpcost). It is preferred that both the gapcost and the jumpcost are positive and that the score matrix has a negative expectation value. An example for a score matrix w with pairings for all 20 standard amino acids is given in Fig. 4. This score matrix is also the basis for the examples given below. The naming of the amino acids conforms to the IUPAC nomenclature.

Let $S = s_1,..., s_n$ denote the candidate sequence and let A be the seed alignment with K rows and m columns, $A = (A_{kj})$ with k <=K and j <=m. The optimal jumping alignment score of the candidate sequence and the seed alignment are to be calculated. It should be noted that the optimal jumping alignment score is unique, whereas there might be more than one alignment obtaining this score. Jumping alignments are local alignments which means that only a segment of the candidate sequence is aligned to a segment of the seed sequences.

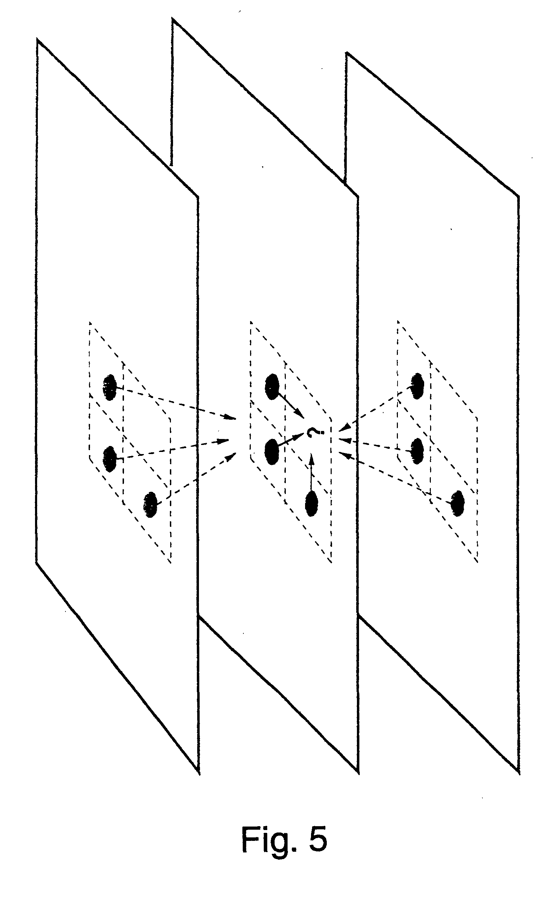

According to the invention, the optimisation problem is solved by an extension of the Smith-Waterman algorithm. Instead of a single two dimensional edit matrix D(i, j), K edit matrices $D_1(i,j),...,D_K(i,j)$ or a three dimensional edit matrix D(i, j, k) are employed, one matrix or "layer" for each sequence in the seed alignment. $D_k(i,j)$ holds the maximal score of all jumping alignments which end with positions $s_i$ and $A_{k,j}$, see Figure 5. For calculating $D_k(i, j)$ one needs to know the value of 3K predecessors cells: 3 predecessor cells in Dk as in the case of pairwise alignment, and 3 additional predecessor cells in each of the K- 1 other edit matrices $D_{k'}$ with k' ≠ k. The maximal entry in the set of all edit matrices gives the optimal jumping alignment score for the candidate sequence and the seed. Obviously, this algorithm runs in $O(nmK^2)$ time and uses $O(nmK)$ space. The same applies if one three dimensional matrix is used. The formulas will change accordingly without changes to the obtained results.

According to the invention, the time complexity can be improved. Since a jump from one sequence to another sequence has the same cost for all alignment sequences, there is no need for computing the best jump individually for each alignment sequence. Instead, the optimal values for the three predecessor cells diagonal (d), vertical (v), and horizontal (h) over all alignment sequences, are pre-computed. This can be done in O(K) time. To be more precise, assume the computation of cell $D_k (i,j)$ for all values of k. Let $k_d$, $k_v$, $k_h$ be the sequences with the best scores in the three predecessor cells of the current cell:

$$k_d = \arg \max_{k' \in \{1,...,K\}} \{D(i\text{-}1, j\text{-}1, k')\}$$

$$k_v = \arg \max_{k' \in \{1,...,K\}} \{D(i\text{-}1, j, k')\}$$

$$k_h = \arg \max_{k' \in \{1,...,K\}} \{D(i, j\text{-}1, k')\}$$

Then, when computing $D_k(i,j)$, there is no need to maximise over all other sequences, but only consider sequences k, $k_d$, $k_v$, and $k_h$. This reduces the time complexity to O(nmK).

Affine gap costs pose the next problem. The algorithm described by Huang et al. can be used as a guideline for calculating optimal jumping alignments with affine gap penalties. Careful consideration is to be given to precise scoring of the affine gaps in the setting of jumping alignments. Gaps that are introduced by the jumping alignment algorithm have to be distinguished from gaps which already exist in the seed alignment. Furtheron, the question how to score gaps in the candidate sequence that run parallel to gaps in the seed alignment has to be answered. The following table gives an example for such gap penalties under different conditions:

| candidate | ***** | *___* | | *___* | ***** |
|---|---|---|---|---|---|
| seed | *___* | ***** | | *...* | *...* |
| gap | | | | | |
| penalty | gapinit + 2gapext | gapinint + 2gapext | 0 | 3gapext | |

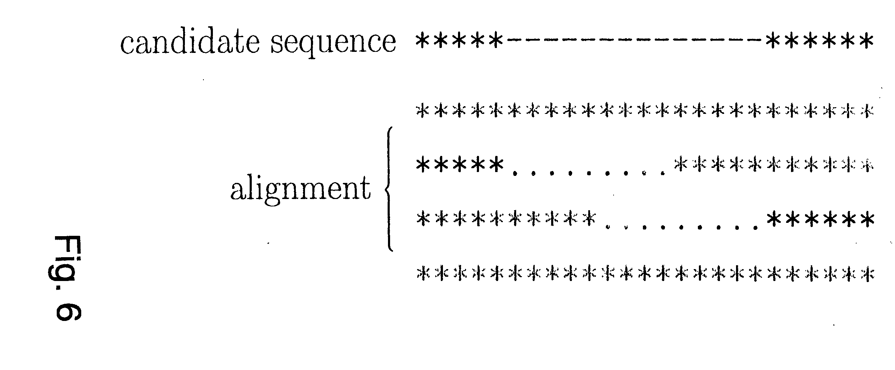

The above table summarises the different types of gaps in the jumping alignment setting, and how they are scored in the inventive process. The full gap penalty only applies if the gap is inserted into the seed alignment (1) or if it is inserted into the candidate sequence and does not run parallel to gaps in the seed alignment (2). A gap in the candidate sequence that runs parallel to a gap in the seed alignment shows that the candidate sequence is "similar" to at least one of the seed sequences and is thus. not penalised (3). Next, one needs to consider the rare case where characters in the candidate sequence run parallel to gap symbol characters in the seed alignment (4). These gaps are penalised only by the gap extension penalty to account for the fact that there are other sequences in the alignment that do contain characters in this region. Finally, there is the jump from a gap in one alignment sequence into a gap in another alignment sequence, as is shown in Figure 6. In this case, the jump cost is applied, but the gap is treated like a single gap so that the gap initiation penalty is imposed only once.

In order to include affine gap costs, similar to Huang et al., the present invention uses auxiliary matrices V and H that hold intermediate score values. To make the inventive method more efficient, the two sequences with the best vertical and horizontal predecessor in the matrices V and H, respectively, are pre-computed:

$$k_V = \arg \max_{k' \in \{1,\dots,K'\}} \{V(i\text{-}1, j, k')\}$$

$$k_H = \arg \max_{k' \in \{1,\dots,K'\}} \{H(i, j\text{-}1, k')\}$$

Then the following recurrences compute the optimal jumping local alignment cost with affine gap costs as defined above.

$$V_k(i, j) = \max \begin{cases} D_k(i-1, j) - \text{gapinit} \\ V_k(i-1, j) - \text{gapext} \\ D_{k_V}(i-1, j) - \text{gapinit} - \text{jumpcost} \\ V_{k_V}(i-1, j) - \text{gapext} - \text{jumpcost} \end{cases}$$

$$H_k(i, j) = \max \begin{cases} D_k(i, j-1) - \text{gapinit} \\ H_k(i, j-1) - G_k(j) \\ D_{k_H}(i, j-1) - \text{gapinit} - \text{jumpcost} \\ H_{k_H}(i, j-1) - G_k(j) - \text{jumpcost} \end{cases}$$

$$D_k(i, j) = \max \begin{cases} 0 \\ D_k(i-1, j-1) + w(s_i, A_{k,j}) \\ D_{k_d}(i-1, j-1) + w(s_i, A_{k,j}) - \text{jumpcost} \\ V_k(i, j) \\ H_k(i, j) \\ D_k(i, j-i) \quad (\text{if} A_{k,j} = \text{gapchar}) \\ D_{k_H}(i, j-1) - \text{jumpcost} \quad (\text{if} A_{k,j} = \text{gapchar}) \end{cases}$$

where

$$G_k(j) = \begin{cases} 0 \quad (\text{if} A_{k,j} = \text{gapchar}) \\ \text{gapext} \quad (\text{otherwise}) \end{cases}$$

Finally, in a further preferred embodiment, the space saving techniques developed by Huang et al. may be applied to the inventive jumping alignment method. This will reduce the space complexity to O((n+m)K), while the time complexity is not increased by the introduction of affine gap costs or by the reduction of the space complexity, and will be O(nmK).

The inventive jumping alignment method is designed for the purpose of searching molecular or other sequence or string containing databases for remote homologues of a given protein family or other similar sequences.

In the field of molecular biology, generally protein superfamilies subdivide into families which are less divergent than the entire superfamily. By a superfamily, a maximal set of homologous sequences is denoted, while the term subfamily denotes a subset of a superfamily with the property that, if one knows one of its members, all other subfamily members can easily be found by standard database search methods such as BLAST (Altschul et al., J. Mol. biol. 215, pgs. 403-410; 1990) or FASTA (Pearson, Methods in Enzymology, 183, pgs. 63-98; 1990). The challenge is to detect new subfamilies of a given superfamily.

In the following illustrative example, a dataset consisting of superfamilies with annotated subfamily structure will be used. However, membership to a superfamily should not only be based on sequence similarity. Such a dependence of annotation and methods creates a "chicken and egg"-problem. If using a test set where superfamily membership is assessed on the basis of sequence similarity that was reported as being significant by some search method, clearly any evaluation procedure based on this data would test for the capability of the applied method to reproduce the method that was used for annotation. The only way to circumvent this problem is to use a database of known homologies which is not based on sequence comparison only.

The SCOP database, as described by Murzin et al., J. Mol. Biol.247, pgs. 536-540; 1995; and Hubbard et al., Nucl. Acids Res., 27, pgs. 254-256; 1999, is such a database. Information relating to SCOP may be found at the URL http://scop.mrc-lmb.cam.ac.uk/scop. The SCOP database classifies protein domains according to the categories "class", "fold", "superfamily", and "subfamily". A SCOP superfamily comprises sequences that might have low sequence similarity, but whose structure and function suggest a common evolutionary origin. Folds and classes are more abstract and contain sequences that have structural similarities, but are not related. SCOP superfamilies are subdivided into subfamilies which are less divergent with respect to sequence, structure, and function. It should be noted that the SCOP's classification into superfamily and subfamily include structural and functional knowledge. This is an important feature to circumvent the "chicken and egg"-problem mentioned above. On the other hand, while being based on different concepts, the SCOP dataset meets the definition of the superfamily-subfamily relation as used in the present invention. SCOP superfamilies are divergent sets of homologous sequences, and a SCOP subfamily consists of sequences with close evolutionary relationships; in general close enough such that if one has detected one member, one can easily find the rest by using this sequence as query in a new search.

The SCOP database of release 37 contains 11822 protein domains. The SCOP database is divided into classes pdb100, pdb95, 90 and 40, according to a standard established by the ASTRAL Compendium for Sequence and Structure Analysis" (URL: http://astral.stanford.edu). The complete database is called pdb100. Furthermore, there is a subset of 2670 sequences that do not share more than 95% sequence similarity (pdb95), one of 2,466 sequences that do not share more than 90% sequence similarity (pd590), and finally pdb40 consisting of 1434 sequences. These partial databases are frequently used to cover the majority of all protein structures while dealing with only a limited amount of data. The number of sequences contained in the subsets and the database changes with the releases of the database.

The evaluation setting in this example is very similar to that of Jaakkola et al. mentioned above. The example's intention is to emulate the discovery of unknown subfamilies. Therefore, one subfamily is split off a SCOP superfamily, thus dividing this SCOP superfamily into two parts, a subfamily which is called excluded subfamily and its complement which is called seed. In the next step, sequences from the seed are used to construct a seed alignment, and then search a database for members of the excluded subfamily. In order to evaluate the performance, one will look for the excluded sequence with the highest score, and count the number of non superfamily members that rank above this sequence.

Let c be the maximal number of false positives that one is willing to accept before one would consider the search to have failed. Next, the minimum of c and the actual count of false positives is designated as the "FP-count with cutoff c".

A SCOP superfamily; divided into an excluded subfamily and a seed shall be called a test set. In total, 80 test sets have been used, including test sets from the same superfamily, but with different excluded subfamilies. These 80 test sets are divided into two pans of 40 test sets each. The first part, the so-called calibration data, is used to choose appropriate parameters for the jumping alignment algorithm. The second part, the so-called evaluation data, is used to compare the performance of the inventive method with the HMMER implementation of the hidden Markov model approach.

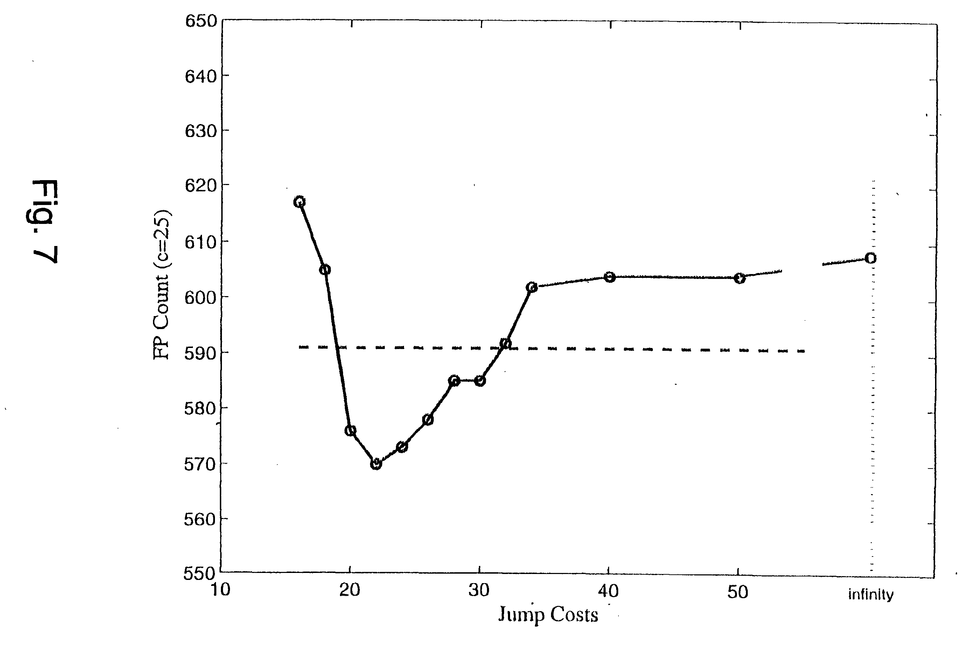

From each seed all sequences that can also be found in the pdb90 database are collected. From these sequences automatically generated multiple alignments using the CLUSTALW program (Higgins et al., Methods Enzymol., 266, pgs. 341-343; 1986) are generated with default parameters. From these aligned sequences are then fetched all sequences that are in the pdb40 database. This yields a multiple alignment consisting of a set of divergent members of the seed. It should be noted that there is no sequence of the excluded subfamily in this alignment and there is no such sequence in the initial larger alignment. These 40 alignments are used as seed alignments for the jumping alignment algorithm and are searched against the pdb95 database. The score matrix according to Fig. 4 is used, while the gap initiation cost are set to 8 and the gap extension cost are set to 4. In this setting, several values for the jump cost parameter are tested. For each of these values the FP-counts (c=25) of all test sets from the calibration data is added. Figure 7 plots the jump costs versus the summed FP-counts which have been summed over all 40 alignments.

The convex shape of the curve can clearly be recognised having a minimum at a jump cost value of 22. The two extreme cases, zero jump costs (purely vertical point of view (data not shown)) and infinite jump costs (purely horizontal point of view) perform worse than intermediate jump costs. This indicates that neither the vertical nor the horizontal point of view is optimal, but a well balanced combination of both. The effect of the gap costs and different score matrices is smaller than that of the jump cost. The choice of a jump cost of 22, however, is optimal for the 40 test sets of the calibration data.

It is instructive to have a look at the two extreme cases, where one chooses either zero or infinite jump costs. Clearly, zero jump costs refer to a purely vertical point of view and infinite jump costs refer to a purely horizontal point of view. A combination is only given for intermediate jump costs. In the evaluations. zero jump costs performed significantly bad (data not shown). There are two explanations for the failure of the algorithm for this choice of parameters. First, the method is a very crude version of a profile search. The established profile search methods are much more elaborate using sequence weighting and Bayesian estimators for the amino acid distribution at each position. Second, the profile approach is mostly restricted to conserved blocks of a protein family. In contrast to zero jumpcosts, infinite jump costs perform surprisingly well. In this case the jumping alignment algorithm is reduced to a very simple method, which is only based on pairwise comparisons.

Ideally, the jump costs should be individually adapted to the concrete conditions of an alignment. In most situations, this will be to complicated or impossible due to a lack of available data. Therefore, the above determined value can be used for data based on the SCOP database without determining the jumpcosts anew. For other sequence data, like DNA comparisons or other text strings, a similar evaluation would need to be made.

The dashed horizontal line in Figure 7 indicates the performance of HMMER on the same data. For an optimal choice of jump costs, the jumping alignment method according to the invention is superior.

Therefore, in another example, the evaluation data are used for comparing the prior art and inventive methods. A principle advantage of hidden Markov models is that the computation time of a database search is independent of the number of sequences used to train the model. Therefore, the entire pdb90-alignments are used to train the hidden Markov models, whereas only the much smaller pdb40-alignments are used as seeds for the inventive jumping alignments. Hence, the hidden Markov models have much more sequences at their disposal than the jumping alignments. However, this still seems to be a fair comparison since the sequences are available. It is the time complexity of the jumping alignment algorithm that makes it impractical to explore this information.

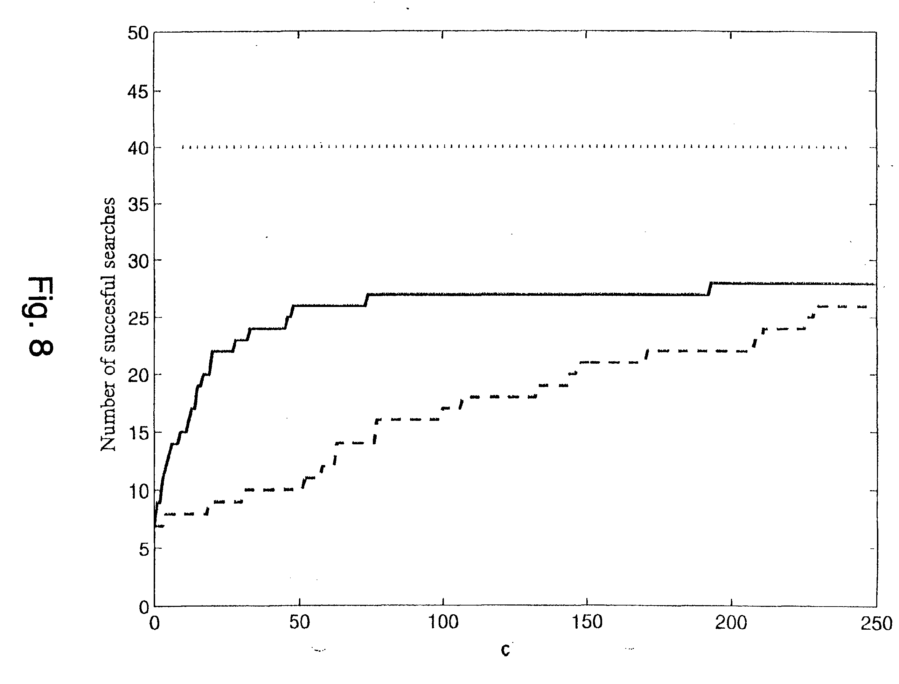

Next, the pdb95 database is searched for the excluded families of the 40 test sets, both using the HMMER algorithm and the inventive method. Figure 8 summarises the results.

The horizontal axis denotes the cutoff c. On the vertical axis, the number of test sets is plotted having no more than c false positives are found before the first excluded sequence is found. The dotted line indicates the total number of test sets in the evaluation setting. First, 12 of the 40 test sets show more than 250 false positives for both methods. This clearly indicates the general limitations of sequence based homology detection. For small values of c, that is, if one is very reluctant to tolerate any false positives, there is no significant difference in the performance of both methods. However, if one increases the cutoff to values higher than 5, the jumping alignments according to the invention find more excluded sequences than the HMMs. For very high cutoff values this difference in performance shrinks again. Obviously, for difficult problems one has to deal with a certain amount of false positives, and it is in this setup of hard problems, where the inventive jumping alignments outperform the hidden Markov models.

In a further example of the invention, the pdb95 database is searched for members of the Calcyclin subfamily (SCOP classification 1.34.1.2) which is a distant relative of a further subfamily of the superfamily Ectatomin (A & B-chains, SCOP classification 1.34.1). For training purposes, the sequences of the other subfamilies of the Ectatomin superfamily are considered to be known. Two database searches are performed.

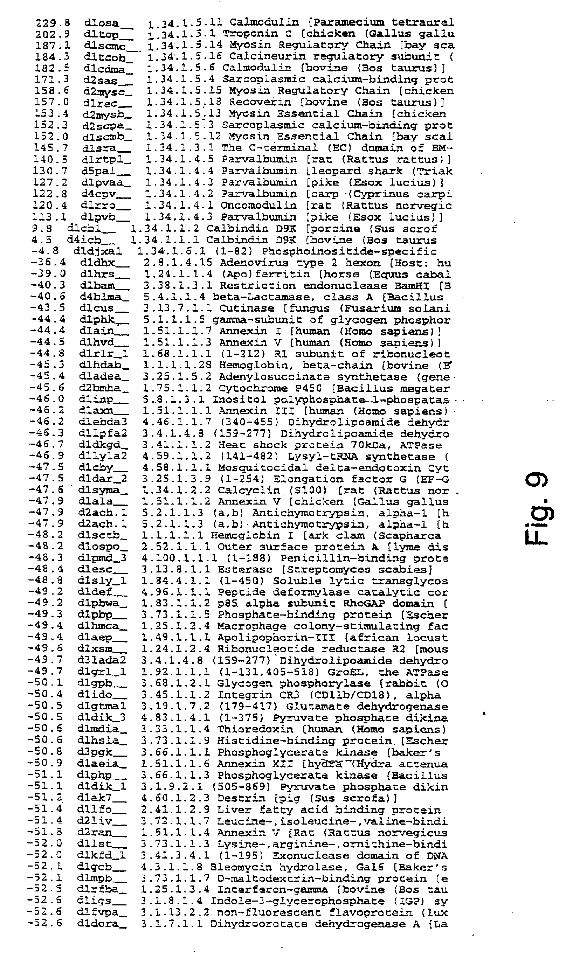

First, a hidden Markov model (HMMER) is trained with the known sequences. Then, all sequences of the database are sorted according to their compatibility with the HMM, as shown in Fig. 9 for the 80 most relevant sequences. As can be seen from Fig. 9, and as was expected, numerous of the sequences used for training are ranked on top of the list. However, the first of the "unknown" Calcyclin sequences only appears at position 42 (Calcyclin S100, 1.34.1.2.2.).

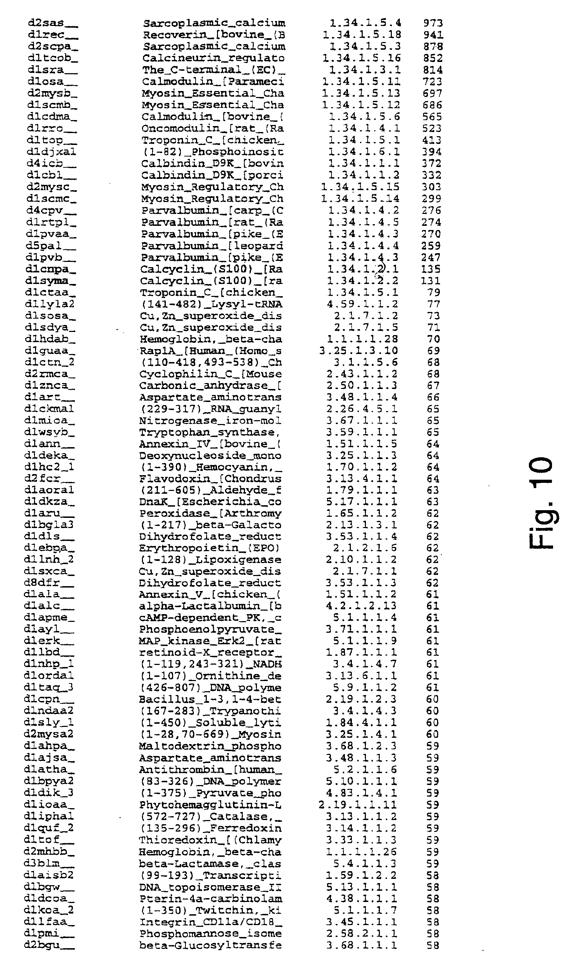

Then, the method according to the invention was applied to the sequence data. As shown in Fig. 10, two Calcyclin type sequences appear directly after the training sequences, with no false positive sequences in between. This clearly shows the superiority of the inventive method over prior art approaches. It should also be noted that, contrary to the hidden Markov model method, no training is necessary.

The invention provides a novel dynamic programming method for detecting remote subfamilies of a string alignment like a given protein superfamily. The principle idea is to exploit both horizontal and vertical information of a multiple-alignment in a well balanced manner.

The inventive jumping alignments balance the horizontal and the vertical information of a multiple sequence alignment. However, this is done locally. When enlarging the alignment, the jumping alignment algorithm according to the invention takes both a horizontal look at the next residues in the reference sequence as well as a vertical look on alternative residues in the current position in other seed sequences.

The inventive concept of the present invention is not to model this correlation statistically, but to deduce the negative effect of hopping by means of a new algorithm. A dynamic programming algorithm is disclosed which locally aligns the candidate sequence to one reference sequence in the multiple alignment, and in addition allows that the reference sequence may be changed within the alignment. This enables the invention to make use of the many alternative residues for a certain column in the alignment. But in order to avoid "wild hopping", each jump is penalised. In this way, the total number of jumps and hence potential noise is reduced.

**Claims**

1. Method for finding a relation between at least one candidate sequence and a number of prealigned seed sequences; wherein the sequences are defined by a consecutive string of characters, each of which belongs to a group of symbols including a gap symbol; with the following step:

   - finding an optimal alignment between the candidate sequence and the prealigned seed sequences by determining for every position of the candidate sequence the best fit with a corresponding position of one of the seed sequences;

   **characterised in that** for determining the optimal individual alignment at each position under evaluation, the following parameters are used:

- the match between the symbol of the candidate sequence and the symbol of the seed sequence at the positions under evaluation;

- the benefit of introducing a gap as a new position into one of the two sequences under evaluation at a position under evaluation; and

- the benefit of jumping from one seed sequence under evaluation to another seed sequence.

2. Method for finding a relation between at least one candidate sequence and prealigned seed sequences especially according to claim 1; wherein the sequences are defined by a consecutive string of characters, each of which belongs to a group of symbols including a gap symbol;
with the following steps:

- setting up a matrix containing in a first dimension the n positions for the characters of the candidate sequence, in a second dimension m positions for the characters of the seed sequences, and in a third dimension K positions for the prealigned seed sequences, wherein K is at least 2, and having $(n + 1) \times (m + 1) \times K$ cells $D(i, j, k)$ with $0 <= i <= n$, $0 <= j <= m$, and $1 <= k <= K$ for holding the score of the best sequence alignment that ends with position i of the candidate sequence and position j of seed sequence k;

- determining a total maximum score $D(i_{max}, j_{max}, k_{max})$ at every cell of the matrix

by recursively computing each $D(i,j,k)$ by setting it as the maximum of the group of possible values given by:

- 0 (zero);

- $D(i-1, j-1, k) + w(s_i, t_{jk})$;

- $D(i-1, j, k) - gapcost$;

- $D(i, j-1, k) - gapcost$

- $D(i-1, j-1, k') + w(s_i, t_{jk}) - jumpcost$

- $D(i-1, j, k') - gapcost - jumpcost$

- $D(i, j-1, k') - gapcost - jumpcost$

wherein

$s_i$ designates the symbol at position i of the candidate sequence,

$t_{jk}$ designates the symbol at position j of seed sequence k;

w is a preset score for every pairwise combination of symbols s and t;

k' is any of K-1 seed sequences except seed sequence k;

a gapcost is a cost for skipping to the next position of one of the two sequences without also stepping to the next position of the other sequence; and

a jumpcost is a cost for jumping to another seed sequence k.

3. Method according to claim 2, **characterised in that** cells $D(1, 1, k)$ are determined by setting D terms used for computing them to zero.

4. Method according to claim 2 or 3, **characterised by** the additional step:

- obtaining the optimal alignment by tracking back through the matrix from the maximum score $D(i_{max}, j_{max}, k_{max})$ along the choices made in the step of maximising the score, until a cell with a value of 0 is reached.

5. Method according to any of claims 2 to 4, **characterised in that** the matrix is comprised of K two dimensional edit matrices with cells $D_k(i,j)$, wherein $1 <= k <= K$.

6. Method according to any of claims 2 to 5, **characterised in that** the gapcost has different values, one of which is used when a gap is first introduce into the alignment, and at least another one which is used when a gap is extended in the alignment.

7. Method according to any of claims 2 to 6, **characterised in that** for the total gapcost of a gap, one of the following values is used:

   - a first gapcost if a gap is to be introduced in either the candidate sequence or the seed sequence;

   - a second gapcost if no gap is introduced in the candidate sequence, even though a gap already exists in the pre aligned sequence; and

   - a gapcost of zero if a gap is introduced in the candidate sequence, provided that a corresponding gap already exists in the pre aligned sequence

8. Method according to claim 7, **characterised in that** the first gapcost is comprised of a gapinit cost, if the gap position is the first one introduced at a particular position i or j of the sequence, and gapext costs, for every consecutive gap position introduced at the following positions of the sequence.

9. Method according to claim 7 or 8, **characterised in that** the second gapcost is comprised of gapext costs for every gap position in a consecutive succession of gap positions in the pre aligned sequence.

10. Process according to any of claims 2 to 9, **characterised in that** the maximum values of

$$D(i-1,j-1,k')$$

$$D(i,j-1,k');$$

and

$$D(i-1,j,k')$$

are pre selected by determining the sequence $k_d$ with the maximum values of the cells $D(i-1,j-1)$, the sequence $k_v$ with the maximum value of the cells $D(i-1,j)$ and the sequence $k_h$ with the maximum value of the cells $D(i,j-1)$ for all sequences k' by the formulas:

$$k_d = \arg \max_{k' \in \{1, \ldots, K\}} \{D(i-1, j-1, k')\}$$

$$k_v = \arg \max_{k' \in \{1, \ldots, K\}} \{D(i-1, j, k')\}$$

$$k_h = \arg \max_{k' \in \{1, \dots, K'\}} \{D(i, j-1, k')\}.$$

**11.** Method according to any of claims 2 to 10, **characterised in that** optimal affine gap costs are calculated by the following additional steps:

- setting up two auxiliary matrices V and H containing in a first dimension n positions for the characters of the candidate sequence, in a second dimension m positions for the characters of the seed sequences, and in a third dimension K positions for the K pre aligned seed sequences, for holding the score of the best sequence alignment that ends with position i of the candidate sequence and position j of the seed sequence k;

- determining the sequence $k_V$ with the maximum values of the cells V(i-1,j), and the sequence $k_H$ with the maximum value of the cells H(i,j-1) for all sequences k' by the formulas:

$$k_V = \arg \max_{k' \in \{1, \dots, K'\}} \{V(i-1, j, k'\}$$

$$k_H = \arg \max_{k' \in \{1, \dots, K'\}} \{H(i, j-1, k'\}.$$

**12.** Method according to claim 11, **characterised by** the following additional step:

- determining a total maximum score $D(i_{max}, j_{max}, k_{max})$ at every cell of the matrix

by recursively computing each V(i,j,k) by setting it as the maximum of the group of possible values given by:

D(i-1,j,k) - gapinit

V(i-1,j,k) - gapext

$D(i-1,j,k_v)$ - gapinit- jumpcost

$V(i-1,j,k_v)$ - gapext - jumpcost;

by recursively computing each H(i,j,k) by setting it as the maximum of the group of possible values given by:

D(i,j-1,k) - gapinit

H(i,j-1,k) - G(j,k)

$D(i,j-1,k_h)$ - gapinit- jumpcost

$H(i,j-1,k_H)$ - G(j, k) - jumpcost;

and

by recursively computing each D(i,j,k) by setting it as the maximum of the group of possible values given by:

- 0 (zero);

- $D(i-1,j-1,k) + w(s_i, A_{k,j})$;

- $D(i-1,j-1,k_d) + w(s_i, A_{k,j})$ - jumpcost;

- $V(i,j,k)$;

- $H(i,j,k)$;

- $D(i,j-i,k)$ if $A_{k,j}$ =gapchar;

- $D(i,j-1,k_h)$ - jumpcost if $A_{k,j}$ =gapchar

wherein $A_{k,j}$ designates the symbol at the j-th position of the seed sequence under evaluation;
gapchar designates the symbol used for indicating a gap;
G(j, k) is 0 if $A_{k,j}$ =gapchar or gapext otherwise; and
gapext is the value used when a gap is extended.

Fig. 1

Fig. 2

Fig. 3

```
C  10
S   0   3
T  -1   1   4
P  -3   0   0   7
A   0   1   1   0   3
G  -3   0  -1  -2   0   6
N  -2   1   0  -2  -1   0   5
D  -3   0  -1  -1  -1   0   2   5
E  -3  -1  -1  -1  -1  -1   0   2   5
Q  -2   0  -1   0  -1  -2   0   0   1   5
H  -1  -1  -1  -1  -2  -2   1   0  -1   2   7
R  -2  -1  -1  -1  -1  -1   0  -1  -1   1   1   6
K  -3  -1   0  -1  -1  -2   1   0   1   1   0   3   5
M  -1  -2   0  -3  -1  -3  -2  -3  -2  -1  -2  -2  -2   6
I  -1  -2   0  -3  -1  -4  -3  -4  -3  -3  -3  -3  -3   2   4
L  -1  -2  -1  -2  -2  -4  -3  -4  -3  -2  -2  -2  -3   2   2   4
V  -1  -1   0  -2   0  -3  -2  -3  -2  -2  -3  -2  -2   1   3   1   4
F  -1  -2  -2  -3  -2  -4  -3  -4  -4  -3  -1  -3  -3   0   0   1   0   7
Y   0  -2  -2  -3  -2  -2  -2  -3  -3  -2   2  -2  -2  -1  -2  -1  -2   4   8
W  -1  -3  -3  -4  -3  -2  -4  -4  -4  -2  -2  -1  -3  -1  -2  -1  -3   1   2  12
    C   S   T   P   A   G   N   D   E   Q   H   R   K   M   I   L   V   F   Y   W
```

# Fig. 4

Fig. 5

Fig. 6

Fig. 7

Fig. 8

```
229.8  d1osa__   1.34.1.5.11 Calmodulin [Paramecium tetraurel
202.9  d1top__   1.34.1.5.1 Troponin C [chicken (Gallus gallu
187.1  d1scmc__  1.34.1.5.14 Myosin Regulatory Chain [bay sca
184.3  d1tcob_   1.34.1.5.16 Calcineurin regulatory subunit (
182.5  d1cdma_   1.34.1.5.6 Calmodulin [bovine (Bos taurus)]
171.3  d2sas__   1.34.1.5.4 Sarcoplasmic calcium-binding prot
158.6  d2mysc_   1.34.1.5.15 Myosin Regulatory Chain [chicken
157.0  d1rec__   1.34.1.5.18 Recoverin [bovine (Bos taurus)]
153.4  d2mysb_   1.34.1.5.13 Myosin Essential Chain [chicken
152.3  d2scpa_   1.34.1.5.3 Sarcoplasmic calcium-binding prot
152.0  d1scmb_   1.34.1.5.12 Myosin Essential Chain [bay scal
145.7  d1sra__   1.34.1.3.1 The C-terminal (EC) domain of BM-
140.5  d1rtpl_   1.34.1.4.5 Parvalbumin [rat (Rattus rattus)]
130.7  d5pal__   1.34.1.4.4 Parvalbumin [leopard shark (Triak
127.2  d1pvaa_   1.34.1.4.3 Parvalbumin [pike (Esox lucius)]
122.8  d4cpv__   1.34.1.4.2 Parvalbumin [carp (Cyprinus carpi
120.4  d1rro__   1.34.1.4.1 Oncomodulin [rat (Rattus norvegic
113.1  d1pvb__   1.34.1.4.3 Parvalbumin [pike (Esox lucius)]
  9.8  d1cbl__   1.34.1.1.2 Calbindin D9K [porcine (Sus scrof
  4.5  d4icb__   1.34.1.1.1 Calbindin D9K [bovine (Bos taurus
 -4.8  d1djxa1   1.34.1.6.1 (1-82) Phosphoinositide-specific
-36.4  d1dhx__   2.8.1.4.15 Adenovirus type 2 hexon [Host: hu
-39.0  d1hrs__   1.24.1.1.4 (Apo)ferritin [horse (Equus cabal
-40.3  d1bam__   3.38.1.3.1 Restriction endonuclease BamHI [B
-40.6  d4blma_   5.4.1.1.4 beta-Lactamase, class A [Bacillus
-43.5  d1cus__   3.13.7.1.1 Cutinase [fungus (Fusarium solani
-44.4  d1phk__   5.1.1.1.5 gamma-subunit of glycogen phosphor
-44.4  d1ain__   1.51.1.1.7 Annexin I [human (Homo sapiens)]
-44.5  d1hvd__   1.51.1.1.3 Annexin V [human (Homo sapiens)]
-44.8  d1rlr_1   1.68.1.1.1 (1-212) R1 subunit of ribonucleot
-45.3  d1hdab_   1.1.1.1.28 Hemoglobin, beta-chain [bovine (B
-45.4  d1adea_   3.25.1.5.2 Adenylosuccinate synthetase [gene
-45.6  d2bmha_   1.75.1.1.2 Cytochrome P450 [Bacillus megater
-46.0  d1inp__   5.8.1.3.1 Inositol polyphosphate-1-phosphatas
-46.2  d1axn__   1.51.1.1.1 Annexin III [human (Homo sapiens)
-46.2  d1ebda3   4.46.1.1.7 (340-455) Dihydrolipoamide dehydr
-46.3  d1lpfa2   3.4.1.4.8 (159-277) Dihydrolipoamide dehydro
-46.7  d1dkgd_   3.41.1.1.2 Heat shock protein 70kDa, ATPase
-46.9  d1lyla2   4.59.1.1.2 (141-482) Lysyl-tRNA synthetase (
-47.5  d1cby__   4.58.1.1.4 Mosquitocidal delta-endotoxin Cyt
-47.5  d1dar_2   3.25.1.3.9 (1-254) Elongation factor G (EF-G
-47.6  d1syma_   1.34.1.2.2 Calcyclin (S100) [rat (Rattus nor
-47.9  d1ala__   1.51.1.1.2 Annexin V [chicken (Gallus gallus
-47.9  d2ach.1   5.2.1.1.3 (a,b) Antichymotrypsin, alpha-1 [h
-47.9  d2ach.1   5.2.1.1.3 (a,b) Antichymotrypsin, alpha-1 [h
-48.2  d1sctb_   1.1.1.1.1 Hemoglobin I [ark clam (Scapharca
-48.2  d1ospo_   2.52.1.1.1 Outer surface protein A [lyme dis
-48.3  d1pmd_3   4.100.1.1.1 (1-188) Penicillin-binding prote
-48.4  d1esc__   3.13.8.1.1 Esterase [Streptomyces scabies]
-48.8  d1sly_1   1.84.1.1.1 (1-450) Soluble lytic transglycos
-49.2  d1def__   4.96.1.1.1 Peptide deformylase catalytic cor
-49.2  d1pbwa_   1.83.1.1.2 p85 alpha subunit RhoGAP domain [
-49.3  d1pbp__   3.73.1.1.5 Phosphate-binding protein [Escher
-49.4  d1hmca_   1.25.1.2.4 Macrophage colony-stimulating fac
-49.4  d1aep__   1.49.1.1.1 Apolipophorin-III [african locust
-49.6  d1xsm__   1.24.1.2.4 Ribonucleotide reductase R2 [mous
-49.7  d3lada2   3.4.1.4.8 (159-277) Dihydrolipoamide dehydro
-49.7  d1grl_1   1.92.1.1.1 (1-131,405-518) GroEL, the ATPase
-50.1  d1gpb__   3.68.1.2.1 Glycogen phosphorylase [rabbit (O
-50.4  d1ido__   3.45.1.1.2 Integrin CR3 (CD11b/CD18), alpha
-50.5  d1gtma1   3.19.1.7.2 (179-417) Glutamate dehydrogenase
-50.5  d1dik_3   4.83.1.4.1 (1-375) Pyruvate phosphate dikina
-50.6  d1mdia_   3.33.1.1.4 Thioredoxin [human (Homo sapiens)
-50.6  d1hsla_   3.73.1.1.9 Histidine-binding protein [Escher
-50.8  d3pgk__   3.66.1.1.1 Phosphoglycerate kinase [baker's
-50.9  d1aeia_   1.51.1.1.6 Annexin XII [hydra (Hydra attenua
-51.1  d1php__   3.66.1.1.3 Phosphoglycerate kinase [Bacillus
-51.1  d1dik_1   3.1.9.2.1 (505-869) Pyruvate phosphate dikin
-51.2  d1ak7__   4.60.1.2.3 Destrin [pig (Sus scrofa)]
-51.4  d1lfo__   2.41.1.2.9 Liver fatty acid binding protein
-51.4  d2liv__   3.72.1.1.7 Leucine-,isoleucine-,valine-bindi
-51.8  d2ran__   1.51.1.1.4 Annexin V [Rat (Rattus norvegicus
-52.0  d1lst__   3.73.1.1.3 Lysine-,arginine-,ornithine-bindi
-52.0  d1kfd_1   3.41.3.4.1 (1-195) Exonuclease domain of DNA
-52.1  d1gcb__   4.3.1.1.8 Bleomycin hydrolase, Gal6 [Baker's
-52.1  d1mpb__   3.73.1.1.7 D-maltodextrin-binding protein [e
-52.5  d1rfba_   1.25.1.3.4 Interferon-gamma [bovine (Bos tau
-52.6  d1igs__   3.1.8.1.4 Indole-3-glycerophosphate (IGP) sy
-52.6  d1fvpa_   3.1.13.2.2 non-fluorescent flavoprotein (lux
-52.6  d1dora_   3.1.7.1.1 Dihydroorotate dehydrogenase A [La
```

**Fig. 9**

| | | | |
|---|---|---|---|
| d2sas__ | Sarcoplasmic_calcium | 1.34.1.5.4 | 973 |
| d1rec__ | Recoverin_[bovine_(B | 1.34.1.5.18 | 941 |
| d2scpa_ | Sarcoplasmic_calcium | 1.34.1.5.3 | 878 |
| d1tcob_ | Calcineurin_regulato | 1.34.1.5.16 | 852 |
| d1sra__ | The_C-terminal_(EC)_ | 1.34.1.3.1 | 814 |
| d1osa__ | Calmodulin_[Parameci | 1.34.1.5.11 | 723 |
| d2mysb_ | Myosin_Essential_Cha | 1.34.1.5.13 | 697 |
| d1scmb_ | Myosin_Essential_Cha | 1.34.1.5.12 | 686 |
| d1cdma_ | Calmodulin_[bovine_( | 1.34.1.5.6 | 565 |
| d1rro__ | Oncomodulin_[rat_(Ra | 1.34.1.4.1 | 523 |
| d1top__ | Troponin_C_[chicken_ | 1.34.1.5.1 | 413 |
| d1djxa1 | (1-82)_Phosphoinosit | 1.34.1.6.1 | 394 |
| d4icb__ | Calbindin_D9K_[bovin | 1.34.1.1.1 | 372 |
| d1cb1__ | Calbindin_D9K_[porci | 1.34.1.1.2 | 332 |
| d2mysc_ | Myosin_Regulatory_Ch | 1.34.1.5.15 | 303 |
| d1scmc_ | Myosin_Regulatory_Ch | 1.34.1.5.14 | 299 |
| d4cpv__ | Parvalbumin_[carp_(C | 1.34.1.4.2 | 276 |
| d1rtp1_ | Parvalbumin_[rat_(Ra | 1.34.1.4.5 | 274 |
| d1pvaa_ | Parvalbumin_[pike_(E | 1.34.1.4.3 | 270 |
| d5pal__ | Parvalbumin_[leopard | 1.34.1.4.4 | 259 |
| d1pvb__ | Parvalbumin_[pike_(E | 1.34.1.4.3 | 247 |
| d1cnpa_ | Calcyclin_(S100)_[Ra | 1.34.1.2.1 | 135 |
| d1syma_ | Calcyclin_(S100)_[ra | 1.34.1.2.2 | 131 |
| d1ctaa_ | Troponin_C_[chicken_ | 1.34.1.5.1 | 79 |
| d1lyla2 | (141-482)_Lysyl-tRNA | 4.59.1.1.2 | 77 |
| d1sosa_ | Cu,Zn_superoxide_dis | 2.1.7.1.2 | 73 |
| d1sdya_ | Cu,Zn_superoxide_dis | 2.1.7.1.5 | 71 |
| d1hdab_ | Hemoglobin,_beta-cha | 1.1.1.1.28 | 70 |
| d1guaa_ | Rap1A_[Human_(Homo_s | 3.25.1.3.10 | 69 |
| d1ctn_2 | (110-418,493-538)_Ch | 3.1.1.5.6 | 68 |
| d2rmca_ | Cyclophilin_C_[Mouse | 2.43.1.1.2 | 68 |
| d1znca_ | Carbonic_anhydrase_[ | 2.50.1.1.3 | 67 |
| d1art__ | Aspartate_aminotrans | 3.48.1.1.4 | 66 |
| d1ckma1 | (229-317)_RNA_guanyl | 2.26.4.5.1 | 65 |
| d1mioa_ | Nitrogenase_iron-mol | 3.67.1.1.1 | 65 |
| d1wsyb_ | Tryptophan_synthase, | 3.59.1.1.1 | 65 |
| d1ann__ | Annexin_IV_[bovine_( | 1.51.1.1.5 | 64 |
| d1deka_ | Deoxynucleoside_mono | 3.25.1.1.3 | 64 |
| d1hc2_1 | (1-390)_Hemocyanin,_ | 1.70.1.1.2 | 64 |
| d2fcr__ | Flavodoxin_[Chondrus | 3.13.4.1.1 | 64 |
| d1aora1 | (211-605)_Aldehyde_f | 1.79.1.1.1 | 63 |
| d1dkza_ | DnaK_[Escherichia_co | 5.17.1.1.1 | 63 |
| d1aru__ | Peroxidase_[Arthromy | 1.65.1.1.2 | 62 |
| d1bgla3 | (1-217)_beta-Galacto | 2.13.1.3.1 | 62 |
| d1dls__ | Dihydrofolate_reduct | 3.53.1.1.4 | 62 |
| d1ebpa_ | Erythropoietin_(EPO) | 2.1.2.1.6 | 62 |
| d1lnh_2 | (1-128)_Lipoxigenase | 2.10.1.1.2 | 62 |
| d1sxca_ | Cu,Zn_superoxide_dis | 2.1.7.1.1 | 62 |
| d8dfr__ | Dihydrofolate_reduct | 3.53.1.1.3 | 62 |
| d1ala__ | Annexin_V_[chicken_( | 1.51.1.1.2 | 61 |
| d1alc__ | alpha-Lactalbumin_[b | 4.2.1.2.13 | 61 |
| d1apme_ | cAMP-dependent_PK,_c | 5.1.1.1.4 | 61 |
| d1ayl__ | Phosphoenolpyruvate_ | 3.71.1.1.1 | 61 |
| d1erk__ | MAP_kinase_Erk2_[rat | 5.1.1.1.9 | 61 |
| d1lbd__ | retinoid-X_receptor_ | 1.87.1.1.1 | 61 |
| d1nhp_1 | (1-119,243-321)_NADH | 3.4.1.4.7 | 61 |
| d1orda1 | (1-107)_Ornithine_de | 3.13.6.1.1 | 61 |
| d1taq_3 | (426-807)_DNA_polyme | 5.9.1.1.2 | 61 |
| d1cpn__ | Bacillus_1-3,1-4-bet | 2.19.1.2.3 | 60 |
| d1ndaa2 | (167-283)_Trypanothi | 3.4.1.4.3 | 60 |
| d1sly_1 | (1-450)_Soluble_lyti | 1.84.4.1.1 | 60 |
| d2mysa2 | (1-28,70-669)_Myosin | 3.25.1.4.1 | 60 |
| d1ahpa_ | Maltodextrin_phospho | 3.68.1.2.3 | 59 |
| d1ajsa_ | Aspartate_aminotrans | 3.48.1.1.3 | 59 |
| d1atha_ | Antithrombin_[human_ | 5.2.1.1.6 | 59 |
| d1bpya2 | (83-326)_DNA_polymer | 5.10.1.1.1 | 59 |
| d1dik_3 | (1-375)_Pyruvate_pho | 4.83.1.4.1 | 59 |
| d1ioaa_ | Phytohemagglutinin-L | 2.19.1.1.1 | 59 |
| d1ipha1 | (572-727)_Catalase,_ | 3.13.1.1.2 | 59 |
| d1quf_2 | (135-296)_Ferredoxin | 3.14.1.1.2 | 59 |
| d1tof__ | Thioredoxin_[(Chlamy | 3.33.1.1.3 | 59 |
| d2mhbb_ | Hemoglobin,_beta-cha | 1.1.1.1.26 | 59 |
| d3blm__ | beta-Lactamase,_clas | 5.4.1.1.3 | 59 |
| d1aisb2 | (99-193)_Transcripti | 1.59.1.2.2 | 58 |
| d1bgw__ | DNA_topoisomerase_II | 5.13.1.1.1 | 58 |
| d1dcoa_ | Pterin-4a-carbinolam | 4.38.1.1.1 | 58 |
| d1koa_2 | (1-350)_Twitchin,_ki | 5.1.1.1.7 | 58 |
| d1lfaa_ | Integrin_CD11a/CD18_ | 3.45.1.1.1 | 58 |
| d1pmi__ | Phosphomannose_isome | 2.58.2.1.1 | 58 |
| d2bgu__ | beta-Glucosyltransfe | 3.68.1.1.1 | 58 |

Fig. 10

**European Patent Office**

**EUROPEAN SEARCH REPORT**

Application Number

EP 00 10 9664

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | MYERS, G. ET AL: "Chaining Multiple-Alignment Fragments in Sub-Quadratic Time" PROC. 6TH. ANNUAL ACM-SIAM SYMPOSIUM ON DISCRETE ALGORITHMS, 22 - 24 January 1995, pages 38-47, XP000952487 USA * page 38, left-hand column, line 1 - page 39, right-hand column, line 13 * | 1 | G06F17/30 |
| A | US 5 873 052 A (SHARAF MUHAMMAD A) 16 February 1999 (1999-02-16) * column 6, line 22 - column 8, line 52 * * column 2, line 46 - column 3, line 59 * | 1 | |
| A | GOLDSTEIN R.A.: "A Bayesian Approach to Sequence Alignement Algorithms for Protein Structure Recognition" PROC. 27TH. HAWAII INTERNATIONAL CONFERENCE ON SYSTEM SCIENCES, VOL. V BIOTECHNOLOGY COMPUTING, 4 - 7 January 1994, pages 306-315, XP000952486 * page 306, left-hand column, line 1 - page 307, right-hand column, line 44 * | 1 | |
| A | EP 0 646 883 A (HITACHI DEVICE ENG ;HITACHI SOFTWARE ENG (JP)) 5 April 1995 (1995-04-05) * page 16, line 56 - page 19, line 38 * * page 10, line 51 - page 11, line 58; figures 4,5A,5B * | 1 | |

TECHNICAL FIELDS SEARCHED (Int.Cl.7)

G06F

-/--

The present search report has been drawn up for all claims

| Place of search | Date of completion of the search | Examiner |
|---|---|---|
| BERLIN | 12 October 2000 | Deane, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 00 10 9664

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 5 577 249 A (CALIFANO ANDREA) 19 November 1996 (1996-11-19) * column 13, line 50 - column 14, line 20 * * abstract; figure 1 * ----- | 1 | |
| | | | TECHNICAL FIELDS SEARCHED (Int.Cl.7) |

The present search report has been drawn up for all claims

| Place of search | Date of completion of the search | Examiner |
|---|---|---|
| BERLIN | 12 October 2000 | Deane, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 152 349 A1

## ANNEX TO THE EUROPEAN SEARCH REPORT
## ON EUROPEAN PATENT APPLICATION NO.

EP 00 10 9664

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2000

| Patent document cited in search report | | Publication date | Patent family member(s) | | Publication date |
|---|---|---|---|---|---|
| US 5873052 | A | 16-02-1999 | AU | 702393 B | 18-02-1999 |
| | | | AU | 6990898 A | 29-05-1998 |
| | | | CA | 2234678 A | 14-05-1998 |
| | | | EP | 1012749 A | 28-06-2000 |
| | | | JP | 2000500896 T | 25-01-2000 |
| | | | WO | 9820433 A | 14-05-1998 |
| EP 0646883 | A | 05-04-1995 | JP | 7093370 A | 07-04-1995 |
| | | | US | 5706498 A | 06-01-1998 |
| US 5577249 | A | 19-11-1996 | EP | 0583559 A | 23-02-1994 |
| | | | JP | 2673091 B | 05-11-1997 |
| | | | JP | 6098770 A | 12-04-1994 |

EPO FORM P0459

For more details about this annex : see Official Journal of the European Patent Office, No. 12/82

29